# EUROPEAN PATENT APPLICATION

(11) **EP 4 226 951 A1**
(43) Date of publication of application: **16.08.2023**
(21) Application number: 21880136.3
(22) Date of filing: 13.10.2021
(51) Int. Cl.: A61L 2/10, C02F 1/32, A61L 9/20

(54) **UVC IRRADIATION CONTAINER**

(30) Priority: 15.10.2020 JP 2020183200
(71) Applicant: Oyama, Nobuo, Yokohama shi, Kanagawa 227-0067 (JP)
(72) Inventor: Oyama, Nobuo, Yokohama shi, Kanagawa 227-0067 (JP)
(74) Representative: Manitz Finsterwald Patent- und Rechtsanwaltspartnerschaft mbB
(86) International application number: PCT/JP2021/037844
(87) International publication number: WO 2022/080398

(57) **Abstract**

[Problem]

To obtain a functional structure of a UVC irradiation container that allows uniform fluid movement velocity throughout the entire area of the container without causing a "flow passage" to form even when the amount of fluid in the UVC irradiation container is greatly increased, thereby enabling uniform UVC irradiation.

[Solution]

A continuous sterilization method in which a rectangular body-shaped UVC irradiation container is properly fitted with dividing walls to form a single fluid flow consisting of multiple layers of horizontally aligned flows over the entire UVC irradiation area of the container, which is then irradiated with UVC from its inner wall side.

## Description

### FIELD OF THE INVENTION

This invention relates to a method for continuous sterilization of air or water by irradiation with ultraviolet-C radiation (hereinafter referred to as "UVC").

### BACKGROUND OF THE INVENTION

As of July 2020, fluid sterilization devices are commercially available that use a single cylindrical UVC lamp in the center of a cylindrical container that is optically shielded from the external environment from the air in the room or the air or water being transported through pipes, and continuously pass the fluid between it and the inner wall of the container, irradiating the fluid with UVC during the passage time. However, such devices are limited to very small treatment capacities due to their low UVC energy utilization efficiency.

In addition, the ability to sterilize the air by generating a fine line of plasma called a streamer and passing the air through it is now commercially available in air conditioners. However, the fraction of air that strikes the fine plasma in the streamer's passage is limited in principle, which is a serious barrier to achieving reliable sterilization.

### RELATED ART

[Non-Patent Literature 1] Y's Company, "UV irradiation water sterilization equipment (piping type)"
[Non-Patent Literature 2] Paper published in the April 23, 2019 issue of Journal of Physics D: Applied Physics, Volume 52, Number 25, "Inactivation of airborne virus using a packed bed on a thermal plasma reactor."

### SUMMARY OF THE INVENTION

### [Technical Problem]

In order to maximize the effective use of the UVC radiation energy for continuous sterilization using a container that acts as a small environment, which is optically shielded from the external environment, for sterilization by UVC irradiation of fluid moving and passing through it (hereinafter referred to as a "UVC irradiation container" or "container"), if the spacing between the inner wall of the UVC irradiation container with the UVC light source attached (hereinafter referred to as the "light source wall") and the inner wall opposite of it (hereinafter referred to as the "wall opposite the light source") is significantly increased to maximize the rate at which the radiated UVC energy strikes the sterilization target in the fluid, is absorbed, and is effectively utilized, then the difference between the velocity of the fluid at the outlet inlet of the container and the average velocity of the entire fluid moving through the container widens significantly. Partial flow occurs in the "flow passage" connecting the inlet and outlet, where the velocity is significantly greater than the majority of the fluid in the container. This causes the remaining majority of the fluid to remain in the container well beyond the required amount of time, thus causing this portion of the fluid to absorb UVC energy well in excess of the amount needed for sterilization, wasting a great deal of energy.

The present invention solves the issue of being able to increase the maximum amount of fluid in the UVC irradiation container for the same UVC irradiation dose without the occurrence of a "flow passage" as described in the paragraph [0005] above, while providing a functional structure that allows for uniform UVC irradiation as the fluid flowing into the container moves and passes through it at the same speed throughout the whole device.

### [Solution to Problem]

As a structure to achieve the functionality described in the paragraph [0006] above, this issue is solved by continuously sterilizing the fluid to be sterilized as it flows into the UVC irradiation area, which is the area inside the UVC irradiation container with a rectangular shape inside as a basic shape, that is optically shielded from the external environment, and has a large amount of fluid inside that gets irradiated with a uniform amount of UVC energy to all parts of the fluid inside without generating a "flow passage" as described above, and continuously flowing out of the container.

The present invention is a solution to the problem described in the paragraph [0007] above, wherein in a container whose basic inner shape is rectangular and all its inner walls have highly UVC-reflective surfaces or are covered with highly UVC-reflective material, all fluid continuously flowing in from one corner forms a layer of flow of a predetermined thickness and flows parallel to a pair of opposing inner walls. The flow direction is changed 180 degrees on the side of the inner wall perpendicular to the direction of the flow, or on the side of the inner wall opposite to such an inner wall, and this is repeated between such pairs of opposite inner walls, so that a single continuous flow consisting of multiple layers of flow side by side is formed in the UVC irradiation area of the container. Either (i) the flow of the continuously flowing fluid in the UVC irradiated zone is irradiated with UVC from one or both sides of such a pair of opposing inner walls, or (ii) from one or both sides of a pair of opposing inner walls parallel to the faces of the transverse layers. The former method is designated as Type 1 and the latter as Type 2.

In the method of optically isolating the UVC irradiation container from the external environment using the method described in the paragraph [0008] above, ducts for moving fluid into and out of the UVC irradiation area are installed inside the UVC irradiation container from a pair of opposite or a pair of mutually orthogonal inner walls of the container. The ducts are installed parallel to and at predetermined intervals from the inner walls of the UVC irradiation container, and fluid inlets and outlets are provided at one end of each duct partition wall that prevents leakage of UVC radiation (hereinafter referred to as "partition walls"). This can provide an effective optical barrier to the external environment. Optical isolation can be improved by using a material with excellent UVC absorption for the inner wall of the duct or by installing a UVC absorption filter inside the duct.

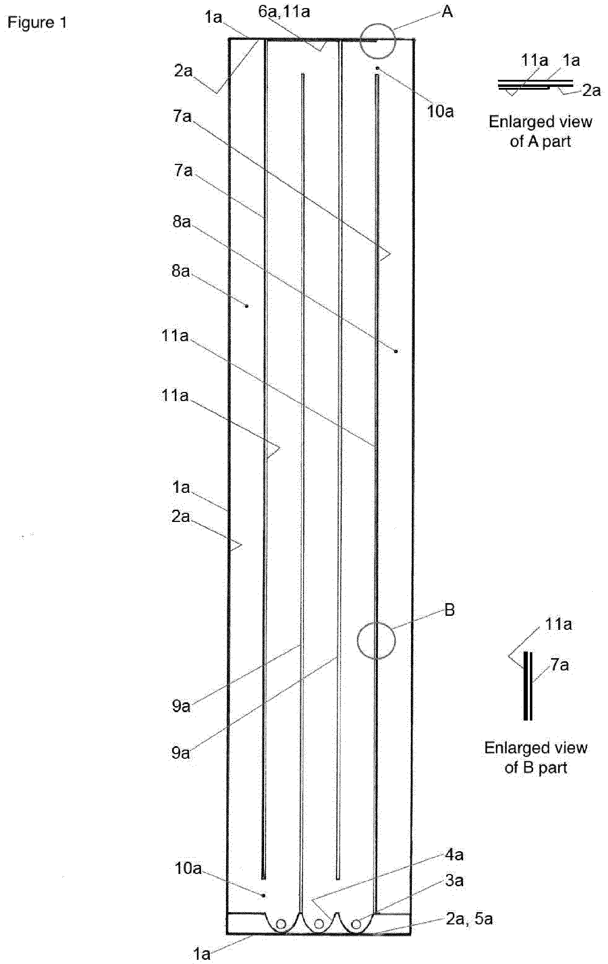

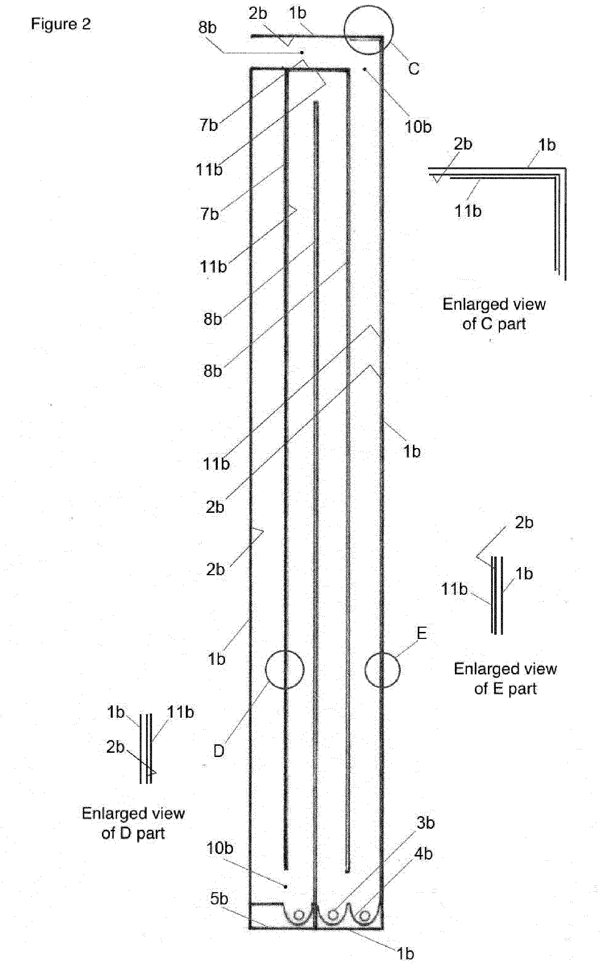

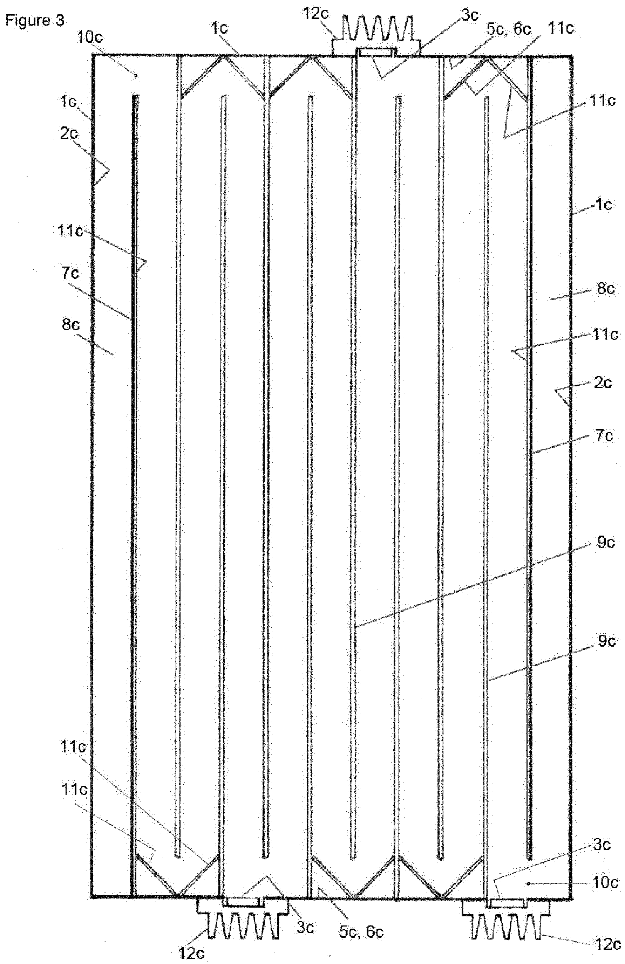

Attached Figs. 1, 2, and 3 are schematic cross sectional views of the three types of UVC irradiation containers of Type 1 described in the paragraph [0008] in a plane parallel to the plane of alignment of the multiple layers of transverse flow formed in the containers, and show a method of forming a single continuous flow consisting of multiple layers of transverse flow of fluid throughout the UVC irradiation area. One or more rectangular dividing walls 9a, 9b, 9c parallel to a pair of opposing inner walls 2a, 2b, 2c that are perpendicular to the alignment surfaces of the plurality of horizontal flow layers described above in Figures 1, 2, and 3, and parallel to the flow direction of such flow layers, between each of such pair of opposing inner walls 2a, 2b, 2c, and with the plurality of dividing walls 9a, 9b, 9c, three of the four end faces or ends of each of the dividing walls 9a, 9b, 9c are adhered directly or indirectly to the inner walls 2a, 2b, 2c at predetermined intervals, including between each other, and the remaining one end face or end has an opening of predetermined width or area between it and the inner dividing walls 9a, 9b, 9c, through which the fluid may pass, or An opening of a predetermined area is formed at the end, and between adjacent dividing walls 9a, 9b, 9c, the pertaining openings are located on opposite sides of each other.

The inlets 10a, 10b, 10c and the outlets 10a, 10b, 10c of the UVC irradiation zone of the fluid container are provided in one corner opposite the opening of dividing walls 9a, 9b, 9c between each of the above pair of opposite inner walls 2a, 2b, 2c and the nearest dividing wall 9a, 9b, 9c, and such outlets 10a, 10b, 10c shall have a shape dimension or area generally the same as the shape dimension or area of the orthogonal transverse section between each of the above pair of opposing inner walls 2a, 2b, 2c and their nearest dividing walls 9a, 9b, 9c, and between dividing walls 9a, 9b, 9c each other.

As a method of optically shielding a UVC irradiation container as described in the paragraphs [0010] and [0011] above against the external environment, it is provided with parallel partition walls 7a, 7b, 7c coming off of the inner walls 2a, 2b, 2c at predetermined intervals on one or both sides of a pair of opposing inner walls 2a, 2b, 2c of the UVC irradiation container on the inside of the container. It is also provided with one or more dividing walls 9a, 9b, 9c parallel to such inner walls 2a, 2b, 2c or 2a, 2b, 2c or partition walls 7a, 7b, 7c as in [0010] above which lie between the inner walls 2a, 2b, 2c or partition walls 7a, 7b, 7c on the opposite side of the container or similarly provided. Also, an inlet 10a, 10b, 10c or outlet 10a, 10b, 10c for fluid is provided leading into or out of the UVC irradiation zone at the end of partition wall 7a, 7b, 7c located opposite the opening of the dividing wall 9a, 9b, 9c nearest to partition wall 7a, 7b, 7c.

This is a fluid inlet 10a, 10b, 10c or outlet 10a, 10b, 10c at one end of partition wall 7a, 7b, 7c that serves as one wall of a duct 8a, 8b, 8c in a container surrounded by a pair of opposing interior walls 2a, 2b, 2c with one opposing end face or end comprising a partition wall 7a, 7b, 7c, the outer inner wall 2a, 2b, 2c thereof, or a dividing wall 9a, 9b, 9c. By meeting the conditions of the outlet 10a, 10b, 10c mentioned in the paragraph [0011] above, a single connected flow comprising multiple layers of side-by-side flow is formed stably throughout the UVC irradiation zone.

In the UVC irradiation container described in [0010] above, the same function as in the paragraph [0012] above is performed by the following structural adjustment depending on the required application conditions. This method has, on the opening side of dividing walls 9a, 9b, and 9c and on the opposite end face side, inside one or both of a pair of opposite inner walls 2a, 2b, and 2c orthogonal to the face of dividing walls 9a, 9b, and 9c, partition walls 7a, 7b, and 7c parallel to such inner walls 2a, 2b, and 2c and having a specified distance therefrom are provided, and in accordance therewith the dimensions of the inner wall can be adjusted such that the relationship between the end face or end of the opening side of the dividing walls 9a, 9b, 9c described in the paragraph [0010] and the inner walls 2a, 2b, 2c is established between such partition walls 7a, 7b, 7c and the opposite interior wall 2a, 2b, 2c or the other partition wall 7a, 7b, 7c with partition walls 9a, 9b, 9c therebetween. An inlet 10a, 10b, 10c or an outlet 10a, 10b, 10c for fluid into or out of the UVC irradiation area is provided at the end face or end side of partition wall 7a, 7b, 7c opposite the opening of partition wall 9a, 9b, 9c nearest to such partition wall 7a, 7b, 7c.

To reduce the ratio of radiated UVC that is absorbed by structures in the UVC irradiation area of the UVC irradiation container, the UVC irradiation area side of all inner walls 2a, 2b, 2c and partition walls 7a, 7b, 7c, are coated with materials with highly UVC reflective surfaces or covered such highly UVC reflective materials.

For the same purpose as in paragraph [0015] above, the dividing walls 9a, 9b, and 9c are produced from sheet materials with UVC highly reflective surfaces, or from sheet, film, or membrane UVC highly transparent materials.

A cavity-shaped mold, in which the outer shape of fluid flow consisting of multiple horizontal layers of flow spread over the entire UVC irradiation area is molded into the inner wall of a cavity made of a highly UVC-permeable polymer such as fluoroplastic, is fitted into the UVC irradiation area and connected to the inlet to and outlet from the UVC irradiation area, thereby obtaining the fluid flow described above. Maintenance of the UVC irradiation container is possible by replacing the cavity-shaped mold.

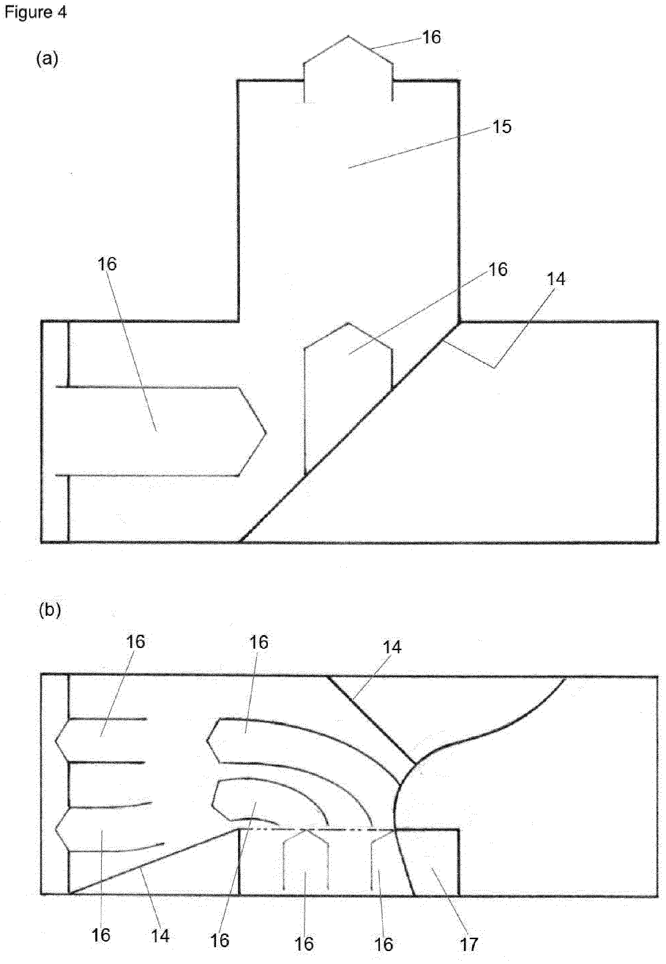

The fluid outflow and intake directions can also be changed in both ducts on the outflow side of the fluid from the UVC irradiation area and on the inflow side of the fluid into the UVC irradiation area, as in Fig. 4.

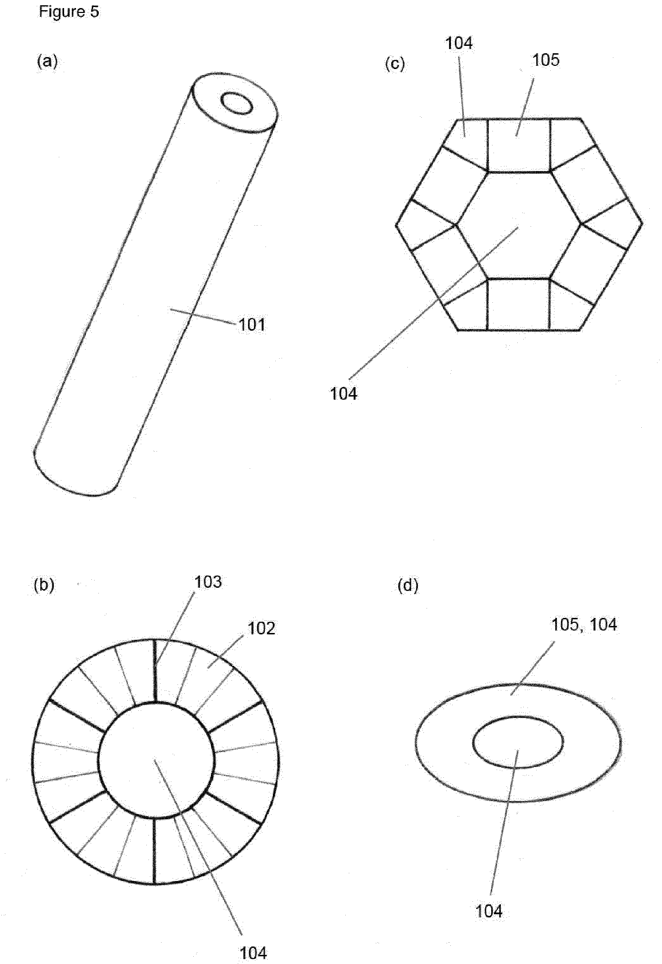

By arranging multiple small units of the UVC irradiation container described above into an annular structure with various cross sections, as shown in Fig.5, it is possible to apply the UVC to various devices and equipment.

### ADVANTAGEOUS EFFECTS OF INVENTION

The two types of UVC irradiation containers outlined in the paragraphs [0007] and [0008] above, respectively, have a basic functional structure that allows the UVC emitted from the light source to travel a large distance with minimal energy loss and to irradiate the entire maximum fluid volume flowing into and out of the container evenly. The basic functional structure of the UVC allows for uniform irradiation of the entire maximum fluid volume flowing into and out of the container with minimal energy loss over a large distance.

### BRIEF DESCRIPTION OF DRAWINGS

Fig. 1 is a cross-sectional view of Type 1-1 UVC Irradiation Container.
Fig. 2 is a cross-sectional view of Type 1-2 UVC Irradiation Container.
Fig. 3 is a cross-sectional view of Type 1-3 UVC Irradiation Container.
Fig. 4 is a cross-sectional view of a duct.
Fig. 5 is an annular arrangement of UVC Irradiation container.

### DESCRIPTION OF PREFERRED EMBODIMENTS

When the target fluid is air, the container proposed in the present invention becomes a UVC irradiation air sterilizer by attaching a cross fan to the fluid outlet of the duct.

With air as the target fluid, and the air outlet of the container's duct is connected to the air intake of an air conditioner, it becomes a sterile air conditioner.

If the target fluid is water, it is used as a sterilization section for water transported through pipes in a water treatment system.

### Example 1

This example pertains to Fig. 1, where the target fluid is air, and is referred to in the paragraph [0010] for Type 1 as defined in the paragraph [0008] of this document.

The outline of the system is as follows: In a stainless steel housing with internal dimensions of 400-mm wide, 950-mm high, and 1200-mm deep, three Philips UVC lamps, G30T8 Bulb 30W UVC lamps, UVC Output: 253.7 nm, are installed as light sources on an internal wall with dimensions of 400-mm wide by 950 mm-high, and UVC is irradiated on the air layer with a thickness of about 80 mm over a total length of about 3.45 m, and the air taken in from the mouth of the air intake duct in the middle of the opposite 950 mm × 1200 mm face is continuously sterilized by a cross fan attached to the air intake duct.

### INDUSTRIAL APPLICABILITY

Air sterilizers with this UVC irradiation container in stand-alone operation are expected to be in strong demand in hospitals, nursing homes, and other facilities.

### REFERENCE SIGNS LIST

1a, 1b, 1c Housing
2a, 2b, 2c Inner walls
3a, 3b, 3c Light source
4a, 4b, 4c Light source reflector
5a, 5b, 5c Light source wall
6a, 6b, 6c Wall opposite the light source
7a, 7b, 7c Partition walls
8a, 8b, 8c Ducts
9a, 9b, 9c Dividing walls
10a, 10b, 10c Inlet to or outlet from UVC irradiation area
11a, 11b, 11c Reflective sheet
12 Heat sink
14 Flow guide plate
15 Duct start section
16 Flow direction
17 Air intake
101 Annular structure
102 Irradiation unit for UVC irradiation container
103 UVC irradiation container partition
104 Space
105 UVC irradiation container

## Claims

1. All of the fluid continuously flowing into the UVC irradiation area, which is within the reach of the UVC radiation of the container, from one corner of the container with a rectangular basic inner shape forms a layer of flow of a predetermined thickness, flows parallel to a pair of opposite inner walls, and (i) changes the direction of flow 180 degrees on the side of the inner wall perpendicular to the direction of the flow, or (ii) makes a similar directional change on the side of the inner wall opposite such inner wall, and repeating this between such a pair of opposite inner walls, with a single continuous flow consisting of multiple layers of horizontally aligned flow is formed over the entire UVC irradiation area of the container, and the continuously flowing fluid in the UVC irradiation area is made to flow over the entire UVC irradiation area created by both sides of such a pair of opposite inner walls. The UVC irradiation container irradiates the flow of continuously flowing fluid passing through the UVC irradiation zone from either or both sides of such a pair of opposing inner walls.

2. The UVC irradiation container of claim 1, wherein a single connected flow consisting of a plurality of transverse flow layers as in claim 1 is irradiated with UVCs from either or both sides of a pair of opposing inner walls parallel to the transverse faces of such plurality of flow layers.

3. For single or multiple rectangular dividing walls parallel to a pair of opposing inner walls of a UVC irradiation vessel whose basic inner shape is rectangular, between each of such pairs of opposing inner walls, or for multiple dividing walls, the UVC irradiation vessel of claims 1 and 2, with a predetermined interval between the opposing inner walls or dividing walls, three of the four end faces or ends of each dividing wall are adhered directly or indirectly to the inner wall, and the remaining one end face or end is provided with an opening of a predetermined width or area through which the fluid passes between it and the inner wall, or an opening of a predetermined area is formed at the end thereof, and between adjacent dividing walls having a structure that makes the shape dimension or area of such outlet approximately the same as the dimensions or area of the orthogonal transverse section between each of the above-mentioned pairs of opposing inner walls and the nearest dividing wall, and mutually between the dividing walls. In addition, the fluid inlet to and outlet from the UVC irradiation area are provided at a corner opposite the opening in the dividing wall between each of the above pairs of opposing inner walls and the nearest dividing wall, and the shape dimensions or area of the outlet are such that the fluid inlet and outlet are located on opposite ends of the dividing wall between the above pairs of opposing inner walls and the nearest dividing wall, and the shape dimensions or area of the outlet are such that the fluid inlet and outlet are located on opposite sides of the dividing wall between the above pairs of opposing inner walls and the nearest dividing wall.

4. The UVC irradiation vessel of claim 1, claim 2, or claim 3, wherein the fluid is provided at one end of the partition wall as an inlet to the UVC irradiation area or an outlet from the UVC irradiation area provided as in claim 3, providing, on one or both sides of a pair of opposing inner walls of the UVC irradiation vessel of claim 3, parallel partition walls at predetermined intervals on the inside of the vessel from such inner walls, and between the inner walls of the opposite side of the vessel or the dividing walls so provided, one or more dividing walls parallel to such inner walls or partition walls are provided as in claim 3, with a fluid inlet to the container or outlet from the container at the end of the partition wall located opposite the opening of the partition wall immediately adjacent thereto, which acts as one wall of a duct in the vessel surrounded by such partition wall, the outer inner wall thereof, and a pair of opposing inner walls with one opposing end face of the partition wall adhering to the other opposing end face of the partition wall.

5. A UVC irradiation vessel of claim 1, claim 2, or claim 3, wherein for the UVC irradiation container of claim 3, wherein on the opening side of the dividing wall and on the opposite end face of the dividing wall, a dividing wall is provided on the inside of one or both of a pair of opposing inner walls orthogonal to the face of the dividing wall, parallel to such inner walls and spaced at a predetermined distance therefrom, wherein the relationship of the end face or end to the inner wall on the opening side of the dividing wall as claimed in claim 3 has dimensions of the dividing wall so as to be established between said partition wall and the inner wall or the other partition wall opposite thereto with a partition wall in between, and an inlet to or outlet from the UVC irradiation zone of the fluid is provided on the end face opposite the opening in the partition wall nearest such partition wall or on the end of the partition wall on the end side.

6. A UVC irradiation container as in claim 1, claim 2, claim 3, claim 4, or claim 5, wherein all inner walls and partition walls within the UVC irradiation area, which is the area within which UVC radiation reaches the UVC irradiation container, are made of materials with highly UVC reflective surfaces, or wherein these are covered with highly UVC reflective materials.

7. A UVC irradiation container as in claim 3, claim 4, claim 5, or claim 6, wherein the face of the dividing wall of claim 3 is made of a UVC highly reflective or highly transmissive material.

8. A UVC irradiation container as in claim 1, claim 2, claim 3, claim 4, claim 5, and claim 6, as well as claim 7 except for UVC irradiation vessels that use UVC highly reflective materials on the face of the dividing wall, wherein a cavity-shaped mold, in which the outer shape of the fluid flow is molded into the inner wall of a cavity made of a highly UVC-permeable polymer such as fluoroplastic, is fitted into the UVC irradiation area and connected to the fluid inlet to and outlet from the UVC irradiation area, as described in claim 1.
